# NEUE EUROPÄISCHE PATENTSCHRIFT

(11) **EP 1 744 812 B2**
(45) Veröffentlichungstag und Bekanntmachung des Hinweises auf die Entscheidung über den Einspruch: **04.06.2014**
(45) Hinweis auf die Patenterteilung: 09.09.2009
(21) Anmeldenummer: 05732659.7
(22) Anmeldetag: 03.05.2005
(51) Int. Cl.: A61N 5/06, A61B 18/22

(54) **LICHTDIFFUSOR UND VERFAHREN ZU DESSEN HERSTELLUNG**
LIGHT DIFFUSER AND PROCESS FOR PRODUCING THE SAME
DIFFUSEUR DE LUMIERE ET PROCEDE DE FABRICATION

(30) Priorität: 03.05.2004 CH 778042004
(43) Veröffentlichungstag der Anmeldung: 24.01.2007
(73) Patentinhaber: Woodwelding AG, 6362 Stansstad (CH)
(72) Erfinder: MAYER, Jörg, CH-5702 Niederlenz (CH); AESCHLIMANN, Marcel, CH-2514 Ligerz (CH); TORRIANI, Laurent, CH-2516 Lamboing (CH); WALT, Heinrich, 8125 Zollikerberg (CH)
(74) Vertreter: Walser, Peter
(86) Internationale Anmeldenummer: PCT/CH2005/000246
(87) Internationale Veröffentlichungsnummer: WO 2005/105208

(56) Entgegenhaltungen:
- EP-A- 0 594 089
- EP-B1- 0 696 185
- WO-A-85/03781
- WO-A1-02/069817
- US-A- 5 209 748
- US-A- 5 304 228
- US-A- 5 530 780
- US-A- 5 695 583
- US-B1- 6 289 150
- US-B1- 6 364 874

## Beschreibung

Die Erfindung betrifft eine Anordnung sowie ein Verfahren zu deren Herstellung. Die Anordnung eignet sich für die diffuse Auslenkung von Licht, das dem Diffusor von einer Lichtquelle oder durch einen Lichtleiter in einer im wesentlichen axialen Richtung zugeführt wird. Die erfindungsgemässe Anordnung eignet sich beispielsweise für die Anwendung in endoskopischen Verfahren, also beispielsweise zum gezielten Einbringen von diffusem Licht in Gewebestrukturen, insbesondere in Knochengewebe, und zum gleichmässigen Beleuchten von Hohlorganen.

Diffuses Licht wird in Gewebestrukturen beispielsweise angewendet in den sogenannten photodynamischen Therapieverfahren, die insbesondere für die Behandlung von Tumor-Erkrankungen bekannt sind. Dem Patienten wird dafür eine Substanz verabreicht, die lichtempfindlich ist und die sich vornehmlich im Tumorgewebe ansammelt Dann wird das Tumorgewebe mit Licht einer spezifischen Wellenlänge belichtet, wodurch die lichtempfindliche Substanz aktiviert und eine chemische Reaktion ausgelöst wird, durch die die Tumorzellen vernichtet werden.

Die Licht-Aktivierung der lichtempfindlichen Substanz leitet die Vernichtung der Tumorzellen ein. Es ist also wichtig, eine spezifische, an die Tumorgrösse angepasste Licht-Dosis in gezielter Weise möglichst homogen in das Tumorgewebe einbringen zu können, was üblicherweise mit Hilfe eines Lichtleiters erreicht wird, wobei das distale Ende des Lichtleiters als Diffusor ausgerüstet ist. Der Diffusor hat die Aufgabe, das sich im Lichtleiter im wesentlichen axial fortpflanzende Licht in möglichst vielen, verschiedenen Richtungen möglichst gleichmässig aus dem Diffusor auszulenken. Der Diffusor wird an das zu belichtende Gewebe heran gebracht oder in das zu belichtende Gewebe eingeführt und über den Lichtleiter mit Licht einer vorgegebenen Wellenlänge versorgt. Durch den Diffusor wird das durch den Lichtleiter zugeführte Licht möglichst homogen in einem Raum verteilt, dessen Form vorteilhafterweise an die Umstände angepasst ist.

Bekannte solche Diffusoren werden hergestellt, indem das distale Ende eines Lichtleiters entsprechend modifiziert wird und/oder indem auf oder an das distale Ende des Lichtleiters ein entsprechend ausgerüstetes Endstück gesetzt wird. Am distalen Ende des Lichtleiters wird also beispielsweise die um die lichtleitende Faser angeordnete Hülle entfernt und die Oberfläche der lichtleitenden Faser wird aufgerauht, angeätzt oder mit geeigneten Werkzeugen bearbeitet zur Erzeugung einer lichtstreuend wirkenden Oberfläche, wie dies beispielsweise in der Publikation FR-2782778 offenbart ist Lichtstreuende Endstücke weisen üblicherweise ein mit Partikeln gefülltes transparentes Material auf (z.B. transparenter Kunststoff mit Partikeln aus Aluminiumoxid oder Titanoxid). Für den Fall, dass die lichtstreuende Wirkung der modifizierten Faseroberfläche und/oder des Endstücks nicht ausreichen, um einen genügenden Anteil des zugeführten Lichtes aus der axialen Richtung auszulenken, wird auch vorgeschlagen, am distalen Ende des Lichtleiters bzw. des Diffusors einen Spiegel anzuordnen, mit dem nicht ausgelenktes Licht in den Diffusorbereich zurück reflektiert wird (z.B. offenbart in US-5695583, US-2002/0094161 und US-5431647).

Die bekannten Lichtdiffusoren stellen also im wesentlichen das distale Ende eines Lichtleiters dar und werden für die medizinische Anwendung mit minimal invasiven Methoden an das zu behandelnde Gewebe heran oder in das Gewebe hinein geführt und nach der Behandlung wieder entfernt. Für die Behandlung wird das proximale Ende des Lichtleiters an eine Lichtquelle angeschlossen, wobei die Lichtquelle beispielsweise ein Laser ist, aber auch das distale Ende eines weiteren Lichtleiters sein kann.

Die oben beschriebenen bekannten Diffusoren werden in relativ aufwendigen Verfahren hergestellt, sie sind also teuer, müssen aber trotzdem meist als Einwegartikel verwendet werden, da sie schwierig zu reinigen und zu sterilisieren sind und für wiederholte Verwendung das Infektionsrisiko klinisch häufig als zu hoch eingeschätzt wird. Ferner müssen sie bei Anwendung in der photodynamischen Therapie in unmittelbare Nähe oder sogar in das zu behandelnde Gewebe geführt und nach der Behandlung von da wieder weggezogen werden, womit immer die Gefahr einer Verschleppung von krankhaften Zellen, beispielsweise von metastasierenden Tumorzellen verbunden ist.

Die Erfindung stellt sich nun die Aufgabe, eine Anordnung mit Lichtdiffusor zu schaffen sowie ein Verfahren zur Herstellung. Der Lichtdiffusor soll sich für verschiedenste, nicht nur medizinische sondern auch technische Anwendungen eignen, insbesondere aber für die oben genannten Anwendungen des Einbringens von diffusem Licht in Knochengewebe (photodynamische Therapie) und für die homogene Beleuchtung von Hohlorganen. Ferner soll das Verfahren zur Herstellung des Lichtdiffusors im Vergleich zur Herstellung von bekannten Lichtdiffusoren einfacher sein und in einfachster Weise eine Anpassung der Geometrie des mit diffusem Licht zu versorgenden Raumes an vorliegende Gegebenheiten ermöglichen.

Diese Aufgabe wird gelöst durch die Anordnung und das Verfahren, wie sie in den Patentansprüchen definiert sind.

Das Verfahren zur Herstellung eines Lichtdiffusors bzw. zum Einbringen von diffusem Licht in Gewebe, insbesondere in Knochengewebe basiert auf dem folgenden Befund: Wenn ein aus einem thermoplastischen Material bestehendes Implantat mit Hilfe von mechanischen Schwingungen, insbesondere mit UItraschall, in Knochengewebe implantiert wird, wie dies beispielsweise in der Publikation WO-02/069817 beschrieben ist, verändert sich seine Oberfläche, insbesondere da, wo diese Oberfläche mit dem Knochengewebe in Berührung steht oder in Berührung gebracht wird, und insbesondere dann, wenn an solchen Stellen Energierichtungsgeber vorgesehen sind. An diesen Stellen verflüssigt sich das thermoplastische Material und wird in Unebenheiten und Poren (trabekuläre Kammern) des Knochengewebes eingepresst, es interpenetriert das Knochengewebe. Unter normalen Implantationsbedingungen erreicht diese Interpenetration zum Beispiel in spongiösem Knochengewebe eine Tiefe, die etwa zwei trabekulären Kammern entspricht. Nach der Wiederverfestigung des thermoplastischen Materials entsteht dadurch eine formschlüssige Verbindung zwischen Implantat und Knochengewebe, die beispielsweise als primäre Stabilisierung des Implantates unmittelbar nach der Implantation ausgenützt wird.

Es zeigt sich nun, dass das in das Knochengewebe eingedrungene, thermoplastische Material dem Implantat auch eine Oberflächenstruktur gibt, die sich optimal eignet, um Licht, das im wesentlichen in axialer Richtung durch die proximale Stirnfläche in ein transparentes solches Implantat eingekoppelt wird, in diffuser Weise aus dem Implantat auszulenken, das heisst, in das das Implantat umgebende Knochengewebe einzulenken. In implantiertem Zustand stellt das Implantat einen hervorragenden Lichtdiffusor dar. Vor der Implantation ist es eine Art Diffusorrohling.

Die durch die Implantation in Knochengewebe mittels mechanischer Vibration erzeugte Oberflächenveränderung, durch die ein entsprechendes Implantat (Diffusorrohling) zum Diffusor wird, entsteht im flüssigen Zustand des Diffusormaterials, so dass die entstehenden Strukturen oberflächenspannungsbedingte Fliessstrukturen sind, die im wesentlichen ein Negativ der porösen Knochenstruktur darstellen, insbesondere also Hinterschnitte aufweise.

An einem stiftförmigen Implantat aus Poly-LDL-Lactid (Länge 25 mm, Durchmesser 3,5 mm), in das von der proximalen Stirnseite mit Hilfe eines Lichtleiters (Durchmesser 0,4 mm) Laserlicht einer Wellenlänge von 625nm eingekoppelt wird, wird am distalen Implantatsende ca. 75% der eingekoppelten Lichtintensität gemessen, was eine sehr anisotrope Lichtverteilung darstellt Wird dasselbe Implantat mittels Ultraschall und ohne Vorbohren in "Sawbone" (glasfaserverstärkter Polyurethanschaum, geschlossenporig), das eine sehr knochenähnliche Struktur hat, eingetrieben, verändert sich die Oberfläche und wird lichtstreuend. Nach dieser Behandlung wird über die ganze, durch die Implantation veränderte Oberfläche eine im wesentlichen gleiche Lichtintensität gemessen (distales Ende: 0,22 W/mm2; Mantelfläche: 0,20 W/mm2). Diese Messungen zeigen, dass die veränderte Oberfläche das eingekoppelte Licht sehr homogen streut, das heisst aus dem Implantat einen sehr guten Diffusor macht.

Der oben beschriebene Befund gilt nicht nur für Knochengewebe sondern ist ohne weiteres auch auf andere, insbesondere künstliche, poröse Materialien übertragbar, wobei solche künstlichen Formmaterialien wie Knochengewebe eine poröse Struktur aufzuweisen haben. Die Poren dieser porösen Strukturen sind vorteilhafterweise von einer Grössenordnung von ca. 0,005 bis 1,0 mm. Ferner muss das Formmaterial derart beschaffen sein, dass seine poröse Struktur durch den beim Einbringen des Diffusorrohlings in das Formmaterial mittels mechanischer Vibration einen für die Verflüssigung und für die Interpenetration genügenden mechanischen Widerstand entgegenbringen kann. Ist dies nicht der Fall, kollabiert die poröse Struktur und es findet keine für die Entstehung der gewünschten Oberflächenstruktur notwendige Interpenetration des porösen Formmaterials statt.

Anstatt einen festen Diffusorrohling mittels mechanischer Vibrationen in Bereichen, in denen der Diffusorrohling mit dem porösen Formmaterial in Kontakt steht, zu verflüssigen und mittels Druck auf den Diffusorrohling in das poröse Formmaterial einzupressen, kann auch ein flüssiges Diffusormaterial in das poröse Formmaterial eingepresst oder eingesaugt werden (z.B. durch Kapillarkraft oder Druckdifferenz). Das flüssige Diffusormaterial wird dann verfestigt durch Abkühlen (z.B. thermoplastische Polymere, Gläser), durch eine entsprechende chemische Reaktion (z.B. vernetzende Harze wie Epoxidharze oder Silikone) oder durch Gelieren (z.B. Gele oder Hydrogele auf der Basis von Polyethylenglykolen, Alginaten, Chitosanen, Collagenen und deren Copolymere oder Blends). Auf diese Weise ist es also nicht nur möglich, viel beliebiger geformte Diffusoren herzustellen als mit dem "Implantationsverfahren" sondern es wird durchaus auch möglich, in einem flexiblen Formmaterial, das nachträglich nicht vom Diffusor entfernt wird, einen gelförmigen, das heisst flexiblen Diffusor zu erstellen, der sich beispielsweise zur Belichtung von Hohlraumwänden eignet, da er sich an verschiedene Hohlraumformen anzupassen vermag oder z.B. bei der Verwendung von resorbierbaren Hydrogelen sogar in einem entsprechenden Hohlraum belassen werden kann. Ein solcher Lichtdiffusor kann beispielsweise für Tumorexcissionswunden nicht nur die Belichtungsfunktion übernehmen sondern nach der Bestrahlung die Wunde auch tamponieren, wobei er dazu vorteilhafterweise in an sich bekannter Weise mit Wirkstoffen wie Cytostatika, antiinflammatorischen Wirkstoffen, Antibiotika oder Wachstumsfaktoren zu weiteren Behandlung des Defektes modifiziert ist.

Ein für die Herstellung des Diffusors geeignetes künstliches, poröses Formmaterial kann derart beschaffen sein, dass es nach der Herstellung des Diffusors beispielsweise durch Auflösen in einem geeigneten Lösungsmittel, durch Ätzen, durch Schmelzen oder Sublimieren vom Diffusor entfernbar ist. Sofern das Formmaterial zumindest lokal selber entsprechende optische Eigenschaften besitzt, kann es aber auch auf der Diffusoroberfläche verbleiben und eine Art von Diffusorkappe bilden, die durch ihre Porosität beispielsweise weiter streuend auf aus dem Diffusor ausgelenktes Licht wirken kann. Eine derartige Diffusorkappe aus dem porösen Formmaterial kann bereits für die Diffusorherstellung eine kappenartige, das heisst relativ dünnwandige Form haben oder sie kann nach der Diffusorherstellung entsprechend bearbeitet werden. Die Diffusorkappe kann aber auch für eine spezifische, nicht-optische weitere Funktion geformt sein oder durch nachträgliche auftragende, umformende oder abtragende Verfahren entsprechend geformt werden. Die-Porosiät des Formmaterials kann homogen sein. Insbesondere für Diffusorkappen mit spezifischen, nicht-optischen weiteren Funktionen mag es vorteilhaft sein, die Porosität inhomogen zu gestalten und funktionsbezogen je nach Stelle der Diffusorkappe zu variieren. So kann eine Diffusorkappe da, wo sie von einem Diffusormaterial interpenetriert werden soll porös sein, während die äussere Kappenoberfläche glatt und porenfrei ist, um beispielsweise in der endoskopischen Anwendung Reibung im Gewebe und Verschmutzung zu minimieren.

Diffusoren, die mit Hilfe eines künstlichen Formmaterials erstellt werden, eignen sich für nichtmedizinische und medizinische Anwendungen, insbesondere aber für die Einbringung von diffusem Licht in Weichgewebe oder in Gewebehohlräume (z.B. Blutgefässe, Atmungswege oder Verdauungstrakt). Dabei wird für die Einbringung des diffusen Lichtes beispielsweise gleich verfahren, wie dies mit Diffusoren gemäss dem Stande der Technik der Fall ist, wobei der Diffusor an einen Lichtleiter oder eine Lichtquelle angekoppelt und für die Anwendung positioniert wird. Dann wird durch den Lichtleiter Licht der gewünschten Wellenlänge in den Diffusor eingekoppelt und durch diesen gestreut und dadurch in das Gewebe eingebracht. Ein besonderer Vorteil bei flexiblen, nach den vorgeschlagenen Verfahren hergestellten Diffusoren besteht darin, dass der Diffusor durch entsprechende aus der Kathetertechnik bekannte Konstruktionen vom Operateur aufgrund der Flexibilität um einen grossen Raumwinkel umgelenkt werden kann, um damit einerseits die Führung des Instrumentes und andererseits eine gezielte Ausleuchtung zu ermöglichen.

Es ist auch möglich, zu streuendes Licht nur in einen Teilbereich des Diffusors einzukoppeln und die restlichen Bereiche für andere Funktionen auszurüsten, wobei diese restlichen Bereiche dann beispielsweise nicht transparent sind.

Die Verwendung von vitalem Gewebe, insbesondere von Knochengewebe, als poröses Formmaterial für die Herstellung des Diffusors aus einem Diffusorrohling bedeutet, dass der Diffusorrohling implantiert wird und die lichtstreuenden Oberflächenstrukturen während der Implantation (in situ) entstehen. Für die Implantation kann im Knochenmaterial vorgängig eine Öffnung (z.B. Bohrung) erstellt werden oder nicht. Beispielsweise kann die kortikale Schicht eines Knochens vorgebohrt und das Implantat in der Bohrung positioniert und von dort mit einer Presskraft und mit gleichzeitiger Vibration in die nicht vorgebohrte Spongiosa eingetrieben werden. Mit einem derartigen, in situ erstellten Diffusor kann in einfachster Weise ein in der Spongiosa lokalisierter Tumor (oder Metastase) belichtet werden. Das Diffusor-Implantat kann für weitere Belichtungen im Knochengewebe verbleiben, wo es gegebenenfalls durch seine intensive Verankerung zusätzlich eine willkommene Verstärkung des durch den Tumor geschwächten Knochengewebes darstellen kann. Das Diffusor-Implantat kann auch aus einem biologisch resorbierbaren, lichtleitenden Material bestehen, so dass es nach seiner Verwendung für die Belichtung des Gewebes nicht entfernt werden muss und allmählich durch regeneriertes Knochengewebe ersetzt wird.

Wenn das Diffusor-Implantat nach der Belichtung am Implantationsort verbleiben soll, ist dafür zu sorgen, dass das proximale Ende des Diffusor-Implantates nicht wesentlich aus dem Knochen vorsteht, dass also sein proximales Ende ausgerüstet ist für den Anschluss an einen Lichtleiter, der wie in bekannten, endoskopischen Verfahren für die Belichtung zu diesem proximalen Ende vorgeschoben wird.

Die wesentlichen Vorteile des in vitalem Knochengewebe durch Implantation erstellten Diffusors gegenüber bekannten derartigen Diffusoren bestehen darin, dass nicht unbedingt vorgebohrt werden muss und dass das Implanat nach der Verwendung des Diffusors für eine Belichtung oder Aktivierung nicht notwendigerweise entfernt oder nicht sofort entfernt werden muss. Dies bedeutet, dass kein Element vom zu behandelnden Gewebe vor oder unmittelbar nach der Behandlung entfernt werden muss und damit die Gefahr der Verschleppung von krankhaften Zellen, z.B. metastasierenden Tumorzellen relevant reduziert wird.

Der Diffusor und das Verfahren zu seiner Herstellung werden im Folgenden im Zusammenhang mit den Figuren im Detail beschrieben. Dabei zeigen:
- **Figur 1**: das Verfahren zur Herstellung des Lichtdiffusors am Beispiel eines Diffusors, der einen etwa zylindrischen Wirkungsbereich haben soll;
- **Figur 2**: einen weiteren beispielhaften Diffusorrohling und den daraus hergestellten, erfindungsgemässen Lichtdiffusor, der einen mehr kugeligen Wirkungsbereich aufweist;
- **Figuren 3 und 4**: Intensitätsprofile von Lichtdiffusoren gemäss Figuren 1 und 2;
- **Figuren 5 und 6**: weitere beispielhafte Ausführungsformen von Diffusorrohlingen für die Herstellung von Lichtdiffusoren mit verschiedenen Wirkungsbereichen;
- **Figur 7**: einen weiteren Lichtdiffusor, der einen für weitere Funktionen ausgerüsteten Diffusorkern aufweist;
- **Figuren 8 und 9**: Varianten der Lichteinkoppelung in einen Lichtdiffusor gemäss Figur 7;
- **Figur 10**: einen weiteren Lichtdiffusor mit einem hohlen Diffusorkem, der für weitere Funktionen ausgerüstet ist;
- **Figuren 11 und 12**: Diffusoren mit Diffusorkappen, die für weitere Funktionen ausgerüstet sind.

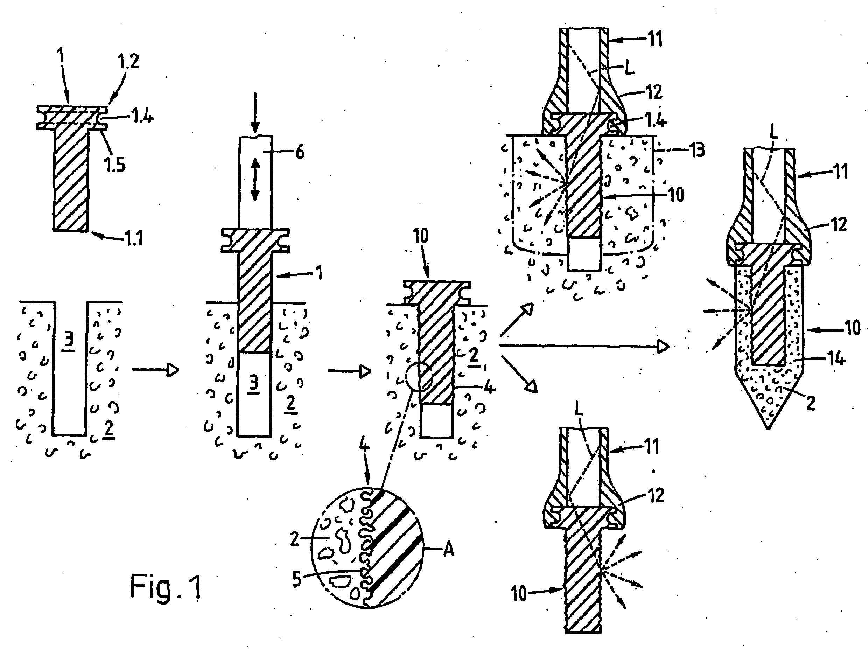

**Figur 1** illustriert das Verfahren zur Herstellung eines Lichtdiffusors mit einer Reihe von Schnittdarstellungen. Dargestellt ist die Herstellung eines beispielhaften Lichtdiffusors, der einen etwa zylinderförmigen Wirkungsbereich haben soll. Wie weiter oben bereits beschrieben, kann der Diffusor in situ in einem Knochen hergestellt werden, oder ex situ mit Hilfe eines künstlichen Formmaterials, das vom Diffusormaterial in einer Grenzschicht interpenetriert wird, wobei das Formmaterial für die Verwendung des Diffusors als Diffusorkappe am Diffusor belassen oder von diesem entfernt werden kann.

Der Diffusorrohling 1, der aus einem geeignet transparenten, thermoplastischen Material (in festem Zustand) besteht, hat beispielsweise eine im wesentlichen zylindrische Form mit einem distalen Ende 1.1 und einem proximalen Ende 1.2, wobei das proximale Ende 1.2 mit einem Mittel zum Ankoppeln eines entsprechend ausgerüsteten distalen Lichtleiterendes 11 ausgerüstet ist, beispielsweise mit einer umlaufenden Nut 1.4.

Im dargestellten Beispiel soll im wesentlichen die ganze Umfangsfläche des Diffusorrohlings 1, nicht aber seine distale Stirnfläche für eine lichtstreuende Funktion strukturiert werden. Die zu strukturierende Oberfläche besteht also aus dem thermoplastischen Material und ist gegebenenfalls zusätzlich mit Energierichtungsgebem ausgerüstet, beispielsweise mit einem Muster von Höckern oder mit axial verlaufenden Rippen (nicht dargestellt). Diejenigen Oberflächen des Diffusorrohlings 1, die nicht für eine lichtstreuende Funktion strukturiert werden sollen, sind vorteilhafterweise poliert, insbesondere die proximale Stirnfläche für die Einkoppelung des zugeführten Lichts und die distale Stirnfläche für die Reflexion von nicht aus dem Diffusor gestreutem Licht. An der distalen Stirnfläche kann der Diffusorrohling 1 auch eine entsprechende spiegelartige Beschichtung aufweisen.

Für die Herstellung des Lichtdiffusors 10 aus dem Diffusorrohling 1 wird in einem porösen Formmaterial 2 eine Öffnung 3 (z.B. Bohrung) erstellt, die derart dimensioniert ist, dass der Diffusorrohling 1 darin vorteilhafterweise mindestens lokal ein leichtes Übermass zum Bohrungsdurchmesser aufweist. Die Bohrungslänge ist grösser als die axiale Länge desjenigen Teils des Diffusorrohlings 1, der in die Bohrung eingebracht werden soll. Damit der Diffusorrohling nicht zu weit in die Bohrung eingebracht werden kann, weist er entsprechende Mittel, beispielsweise einen proximalen Kragen 1.5 auf.

Der Diffusorrohling 1 wird in der Bohrung 3 des porösen Formmaterials 2 positioniert und dann beispielsweise mit Hilfe einer Sonotrode 6, die mit Ultraschallschwingungen angeregt wird, in die Bohrung 3 gepresst. Dabei verflüssigt sich das thermoplastische Material da, wo es mit dem porösen Formmaterial 2 in Berührung steht, und insbesondere da, wo Energierichtungsgeber (nicht dargestellt) aus dem thermoplastischen Diffusormaterial mit dem porösen Formmaterial 2 in Berührung stehen und durch die mechanische Vibration im Diffusormaterial dadurch Spannungskonzentrationen entstehen. Das verflüssigte Diffusormaterial wird in die Poren des porösen Formmaterials 2 eingepresst und interpenetriert das poröse Formmaterial in einer Grenzschicht 4, die vorteilhafterweise eine Dicke von ca. 0,02 bis 1,0 mm aufweist. Dadurch entsteht nach einer Wiederverfestigung des Diffusormaterials die lichtstreuende Oberflächenstruktur 5, wie sie im Detail A dargestellt ist, und wird der Diffusorrohling 1 zum Diffusor 10. Die erstellte Oberflächenstruktur 4 entspricht im wesentlichen der Porenstruktur des porösen Formmaterials 2 bzw. einem abgegossenen Negativ davon, das heisst, sie weist hinterschnittene und, wegen ihrer Entstehung im flüssigen Zustand des Diffusormaterials, oberflächenspannungsbedingte Formen auf.

Wie rechts in der Figur 1 dargestellt ist, wird der Diffusor 10 für seine Anwendung mit Licht L versorgt, indem an seinem proximalen Ende ein distales Lichtleiterende 11 angekoppelt wird, beispielsweise durch Einschnappen eines entsprechenden Kopplungsstückes 12 in die Nut 1.4. Solche Kopplungen gehören zum Stand der Technik und sind deshalb hier nicht näher beschrieben.

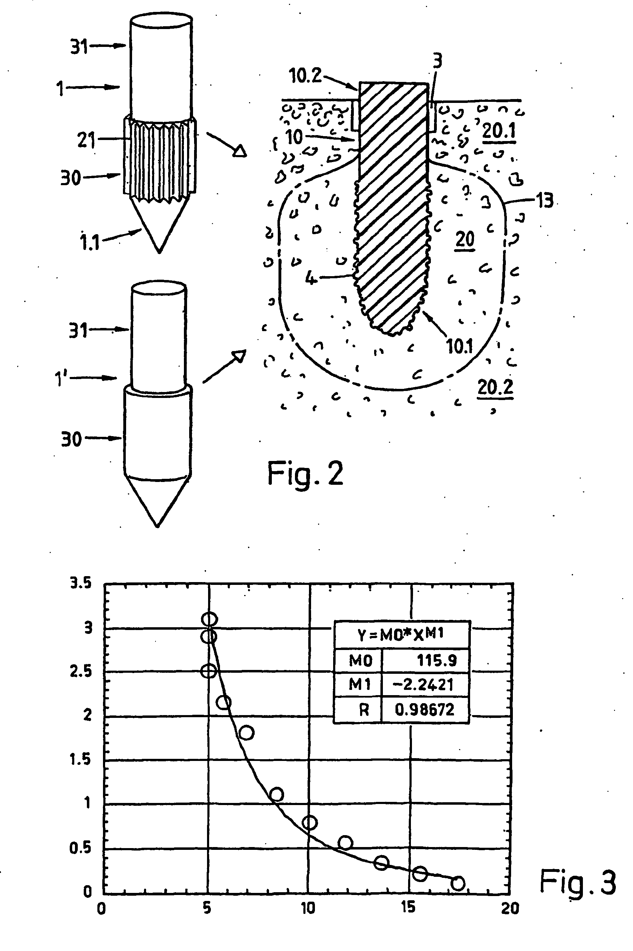

Der Diffusor 10 kann für seine Belichtungsfunktion im porösen Formmaterial 2 bleiben und zum Einbringen von diffusem Licht in dieses Formmaterial dienen, beispielsweise als Belichtungsimplantat in Knochengewebe, wie dies rechts oben in der Figur 1 dargestellt ist. Der Diffusor streut das eingekoppelte Licht in einer sehr homogenen Weise in einem etwa zylinderförmigen Wirkungsbereich, wie er durch die strichpunktierte Linie 13 angedeutet ist (siehe auch Figur 3).

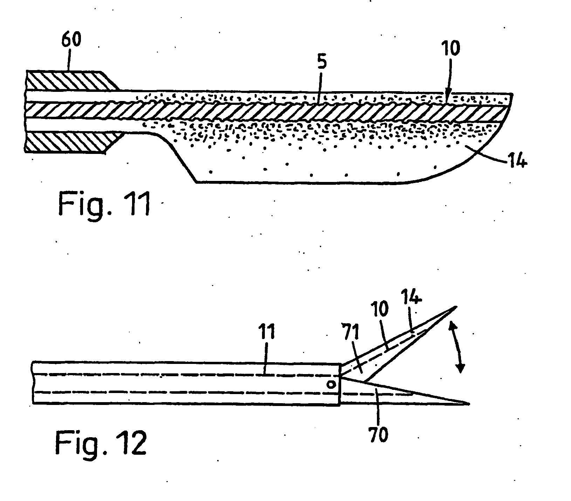

Andererseits kann das poröse Formmaterial 2 (in diesem Falle notwendigerweise transparent) auch eine Diffusorkappe 14 bilden (Figur 1, rechts Mitte). Ein solche Diffusorkappe schützt den Diffusor und kann ferner beispielsweise für eine weitere Streuung des aus dem Diffusor 10 ausgekoppelten Lichts oder für weitere, nicht optische Funktionen ausgerüstet sein. Die Diffusorkappe kann zusätzlich für weitere lichtleitende, ablenkende, abschirmende, fokussierende oder auch filternde Funktionen ausgerüstet sein, wie dies aus dem Stand der Technik hinreichend bekannt ist. Zusätzlich zu ihren optischen Funktionen kann die gegebenenfalls entsprechend nachbearbeitete Diffusorkappe ein Instrument oder Instrumententeil darstellen (siehe Figuren 11 und 12).

Das poröse Formmaterial 2 kann vom Diffusor 10 abgetrennt werden, so dass die lichtstreuende Oberflächenstruktur 4 das einzige lichtstreuende Mittel des Diffusors 10 darstellt (Figur 1, rechts unten).

Für die Ausführungsform des Verfahrens gemäss Figur 1 wird das Diffusormaterial derart ausgewählt, dass der Diffusorrohling 1 eine genügende mechanische Stabilität aufweist, um in die Bohrung 3 gepresst zu werden. Für eine möglichst energiesparende Herstellung, die insbesondere für eine Durchführung in vitalem Knochengewebe auch schonend ist, wird das Diffusormaterial derart gewählt, dass es die mechanische Vibration möglichst wenig dämpft (Elastizitätsmodul grösser als 0,5 GPa).

Transparente oder ausreichend transparent verarbeitete thermoplastische Diffusormaterialien, die sich für in Knochengewebe implantierbare Diffusorrohlinge eignen, sind beispielsweise die biologisch resorbierbaren Polymere auf Milch- und/oder Glukolsäurebasis (PLA, PLLA, PGA, PLGA etc), insbesondere Poly-LDL-Lactid (z.B. erhältlich von Böhringer unter dem Handelsnamen Resomer LR708) oder Poly-DL-Lactid (z.B. erhältlich von Böhringer unter den Handelsnamen Resomer R208) oder die ebenfalls resorbierbaren Polyhydroxyalkanoate (PHA), Polycaprolactone (PCL), Polysaccharide, Polydioxanone (PD), Polyanhydride, Polypeptide oder entsprechende Copolymere oder die nicht resorbierbaren Polyolefine (z.B. Polyethylen), Polyacrylate, Polymetacrylate, Polycarbonate, Polyamide, Polyester, Polyurethane, Polysulfone, Polyphenylsufide oder Flüssig-Kristall-Polymere (liqid cristal polymers oder LCPs), Polyacetale, halogenierte Polymere, insbesondere halogenierte Polyolefine, Polyphenylsulfide, Polysulfone, Polyether oder entsprechende Copolymere und Mischpolymere

Das poröse Formmaterial 2 wird derart gewählt, dass seine Porenstruktur in Kontakt mit dem verflüssigten Diffusormaterial stabil bleibt, aber von diesem Material interpenetriert werden kann. Ein künstliches, poröses Formmaterial weist eine für die Interpenetration geeignete Porosität auf, wobei dies eine offene Porosität ist oder eine geschlossene Porosität mit unter den Verfahrensbedingungen durchbrechbaren Zwischenwänden. Die Poren liegen vorteilhafterweise in einem Grössenbereich von 0,01 bis 1,0 mm. Porengrössen und Porenverteilungen können auch Gradienten aufweisen, beispielsweise zur Erzeugung von fraktalen Oberflächengeometrien oder zur Erzeugung von Diffusorkappen mit einer glatten, porenfreien Oberfläche.

Beispiele für künstliche, poröse Formmaterialien, die als Diffusorkappen am Diffusor verbleiben und weitere Funktionen übernehmen sollen, sind beispielsweise Gläser (Sinterglas, Schaumglas), amorphe Keramiken oder Keramiken mit einem hohen Gehalt an Glasphasen (oxidische Keramiken wie z.B. Aluminiumoxid oder Titanoxid oder nicht oxidische wie z.B. Nitride), dotierte Keramiken (z.B. für weitere optischphysikalische Funktionen wie z.B. Filter oder Anregung von Fluoreszenz) oder amorphe oder weitgehend amporh hergestellte, thermoplastische oder vernetzte Polymere. Zur Erzeugung von porösen Formen der genannten Materialien werden an sich bekannte Methoden verwendet wie z.B. Schäumverfahren, Vakuumverfahren, Leachingverfahren, Sinterverfahren oder Entmischungsverfahren.

Soll das poröse Formmaterial nach dessen Erstellung vom Diffusor abgetrennt werden, hat es einen tieferen Schmelzpunkt als das Diffusormaterial und wird mittels Wärme abgetrennt oder es ist in einem Lösungsmittel löslich, in dem das Diffusormaterial nicht löslich ist, und wird mittels Lösungsmittel abgetrennt. Weitere geeignete Trennverfahren sind Ätzprozesse oder Sublimations- oder Verdampfungstechniken. So kann beispielsweise Schaumgips als poröses Formmaterial mit Hilfe einer schwachen Säure (Lösungsmittel) von einem Diffusor aus einem amorphen Polymer weggelöst werden oder ein sehr natriumhaltiges Glas (z.B. Wasserglas) mit Wasser.

In der Figur 1 dargestellte Diffusorrohlinge und Diffusoren haben eine kreiszylindrische Form. Dies ist selbstverständlich für die Erfindung keine Bedingung. Diffusorrohling und Diffusor können in derselben Weise einen beliebigen Querschnitt aufweisen und sich beispielsweise gegen das distale Ende kontinuierlich oder stufenweise verjüngen.

**Figur 2** zeigt einen weiteren Diffusorrohling 1 und den daraus beispielsweise in einem Knochen 20 (poröses Formmaterial) in situ hergestellten Lichtdiffusor 10. Der Diffusorrohling 1 gemäss Figur 2 hat ein distales Ende 1.1, das spitz ist, und nur ein distaler Bereich 30 seiner Mantelfläche ist für die Erstellung der lichtstreuenden Oberflächenstruktur 4 mit vorstehenden Energierichtungsgebern 21 (z.B. axial verlaufende Rillen) versehen, während der proximale Bereich 31 der Mantelfläche beispielsweise poliert ist oder eine spiegelähnliche Beschichtung aufweist.

Für die Implantation des Diffusorrohlings 1 wird beispielsweise in der kortikalen Schicht 20.1 des Knochens 20 eine entsprechende Öffnung 3 erstellt, die vorteilhafterweise etwas grösser ist als der Querschnitt des Diffusorrohlings und in die der Diffusorrohling 1 mit seinem distalen Ende 1.1 voran positioniert wird. Das spitze, distale Ende 1.1 des Diffusorrohlings 1 wird dann mit Hilfe von Druck und mechanischer Vibration in den spongiösen Knochen 20.2 getrieben, wobei im Bereiche des distalen Endes 1.1 und im Mantelflächenbereich 30 das Diffusormaterial verflüssigt und in die Porenstruktur der Spongiosa eingepresst wird. Es entsteht ein Diffusor 10 mit einem distalen Diffusorteil 10.1 und einem proximalen Lichtleiterteil 10.2.

Offensichtlich kann die Tiefe des Diffusorteils im Knochen vorgegeben werden durch die Wahl der axialen Länge des Diffusorrohlings 1 und der axialen Länge des nicht mit Energierichtungsgebem ausgestatteten Mantelflächenbereichs 31. Die Form des Wirkungsbereiches des Diffusors 10 gemäss Figur 2 ist je nach axialer Länge des mit Energierichtungsgebem 21 ausgestatteten Oberflächenbereichs 30 kugelig oder kugelig/zylindrisch (strichpunktierte Linie 13).

Dank seines proximalen, lichtleitenden Teils eignet sich der Diffusorrohling 1 gemäss Figur 2 insbesondere als Belichtungsimplantat für die photodynamische Behandlung von Tumoren oder Metastasen im Knocheninnem. Der Diffusorrohling 1 ist dabei in seiner Länge an die Tiefe des zu behandelnden Bereichs im Knochen angepasst, die Länge des mit Energierichtungsgebem 21 versehenen Oberflächenbereichs 30 an die Grösse des zu behandelnden Knochenbereichs. Der Diffusorrohling 1 wird dann von der Knochenoberfläche in den Knochen getrieben, bis sein distales Ende im zu behandelnden Knochenbereich positioniert ist und dadurch aus dem Diffusorrohling ein Diffusor geworden ist. Dann wird ein distales Lichtleiterende oder eine Lichtquelle an das proximale Ende des Diffusors angeschlossen und der zu behandelnde Knochenbereich wird belichtet.

Offensichtlich muss der zu behandelnde Bereich für die Belichtung nicht geöffnet und mit keinem Werkzeug in Berührung gebracht werden, wodurch die Gefahr einer Verschleppung von krankhaften Zellen aus diesem Bereich gegenüber Belichtungsmethoden gemäss dem Stande der Technik relevant verkleinert wird.

Je nach Diffusormaterial kann es auch genügen, den distalen Bereich (Oberflächenbereich 30) eines Diffusorrohlings 1' (in Figur 2 unter dem Diffusorrohling 1 dargestellt) nicht mit Energierichtungsgebern 21 auszurüsten, sondern ihm einen gegenüber dem proximalen Oberflächenbereich 31 leicht grösseren Querschnitt zu geben, derart, dass die Oberfläche des distalen Bereichs 30 über die Oberfläche des proximalen Bereichs 31 leicht vorsteht und dadurch in einer Bohrung 3 in intensiveren Kontakt mit dem Knochengewebe 20 kommt als die weiteren Oberflächenbereiche 31, in denen keine lichtstreuende Oberflächenstruktur erstellt werden soll.

Wie bereits im Zusammenhang mit der Figur 1 beschrieben, ist es natürlich auch für die Verfahrensvariante gemäss Figur 2 möglich, ein künstliches Formmaterial zu verwenden und dieses danach entweder auf dem Diffusor als Diffusorkappe zu belassen oder zu entfernen. Insbesondere eignet sich die in Figur 2 dargestellte Verfahrensvariante für die Verwendung eines flüssigen Diffusormaterials. Das flüssige Diffusormaterial wird in eine Form gepresst oder gesaugt (Druckreduktion auf der Formaussenseite), wobei die Form aus dem porösen Formmaterial besteht oder eine Innenbeschichtung aus dem porösen Formmaterial aufweist. Dabei interpenetriert das Diffusormaterial das poröse Formmaterial im Bereiche einer Grenzschicht. Das flüssige Diffusormaterial in der Form und in der Grenzschicht wird dann ausgehärtet durch beispielsweise Abkühlen, Polymerisation oder Gelieren, wodurch ein erfindungsgemässer Lichtdiffusor entsteht, der in der weiter oben für den aus dem Diffusorrohling hergestellten Lichtdiffusor beschrieben Weise weiterverwendet wird.

Als giessfähige Diffusormaterialien können (z.B. chemisch, thermisch oder durch Strahlung) vernetzbare Polymere, wie beispielsweise Silikone, Polyurethane, Epoxidharze oder Polyesterharze verwendet werden. Ebenso eignen sich thermoplastische Polymere, Gele (z.B. PEG, PHEMA, Acrylate, Sacharide, Alginate, Chitosane, oder Copolymere und Mischungen von Alginaten und Chitosanen,), Gläser, Glaskeramiken oder oxidische und nicht oxidische Keramiken mit einem hohen Anteil amorpher Phase. Den Giessmassen können nach dem Stand der Technik zusätzliche streuende Hilfsstoffe wie Titanoxide, Glimmer etc. beigegeben werden.

Als entfernbares, poröses Formmaterial zur Herstellung eines Diffusors aus einem gelierenden Diffusormaterial kann beispielsweise ein Wood'sches Metall verwendet werden. Solche Metalle können bei sehr tiefen Temperaturen gesintert und nach der Erstellung des Diffusors bei Temperaturen, die nur wenig über der Umgebungstemperatur liegen, vom Gel abgeschmolzen werden. Alternativ kann durch Entfernen des Lösemittels im Gel rsp. durch trocknen des Gels und damit verbundene Volumenreduktion der Diffusor aus der Form entfernt werden.

**Figur 3** zeigt ein Intensitätsprofil, das an einem Diffusor gemäss Figur 1 gemessen wurde. Der Diffusor wurde hergestellt dadurch, dass ein stiftförmiger Diffusorrohling (Länge 25mm, Durchmesser 3,5mm) aus Poly-LDL-Lactid mittels Ultraschall (Branson Handgerät, 20kHZ) in einen entsprechend vorgebohrten, spongiösen Knochen (Femur eines Schafes) implantiert wurde. Die Bohrung hatte eine Tiefe von mehr als 12 mm und das Implantat wurde in eine Tiefe von 12 mm, also nicht bis ans Bohrungsende vorgetrieben. Danach wurde über eine lichtleitende Faser (Durchmesser 400µm) Laserlicht einer Wellenlänge von 625nm (Leistung 0,5W) durch die proximale Stirnfläche in das Implantat eingekoppelt und mit Hilfe eines Silikon-Detektors (Durchmesser 7,9mm) an verschiedenen Stellen des Knochenstücks die Lichtintensität gemessen.

Das in der Figur 3 dargestellte Diagramm zeigt die gemessene Lichtintensität [mW] als Funktion des Abstandes von der Diffusoroberfläche [mm]. Der Fit mit einer exponentiell abfallenden Kurve ergibt einen Exponenten von etwa -2,2, der auf einen durch den Diffusor beleuchteten Raum mit einer mehr zylindrischen (theoretischer Exponent = -2) als kugeligen (theoretischer Exponent = -3) Form schliessen lässt.

Die gemessenen Lichtleistungen zeigen, dass es mit einem Implantat von 3.5 mm Durchmesser bei ca. 15 Min. Bestrahlungszeit möglich ist, ein Knochenvolumen von ca. 1.5 cm Durchmesser mit einem Energieeintrag von 10J zu versorgen, was für eine zytotoxische photodynamische Therapie ausreichend ist.

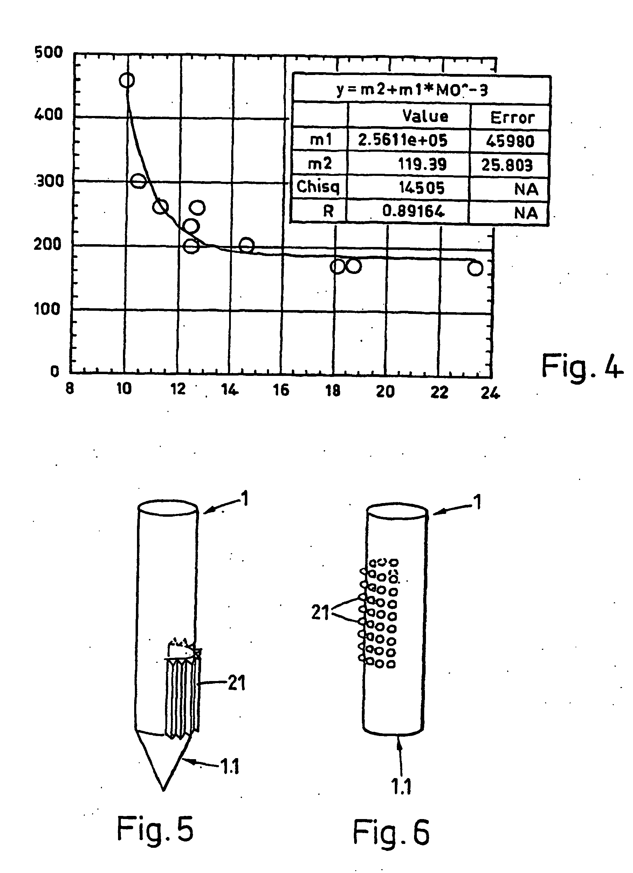

**Figur 4** zeigt ein Intensitätsprofil, das an einem Diffusor gemäss Figur 2 gemessen wurde. Der Diffusor wurde hergestellt dadurch, dass ein stiftförmiger Diffusorrohling (Länge 25mm, Durchmesser 3,5mm) aus Poly-LDL-Lactid mittels Ultraschall (Branson Handgerät, 20kHZ) ohne Vorbohren in ein Stück "Sawbone" (glasfaserverstärkter Polyurethanschaum) bis zu einer Tiefe von 12mm eingepresst wurde. Danach wurde über eine lichtleitende Faser (Durchmesser 400µm) Laserlicht einer Wellenlänge von 625nm (Leistung 0,5µW) durch die proximale Stirnfläche in das Implantat eingekoppelt und mit Hilfe eines Fiber-Detektors (Durchmesser 200µm) an verschiedenen Stellen des Sawbone-Stücks die Lichtintensität gemessen.

Das in der Figur 4 dargestellte Diagramm zeigt die gemessene Lichtintensität [counts] als Funktion des Abstandes von der Diffusoroberfläche [mm]. Der Fit mit einer exponentiell abfallenden Kurve mit einem Exponenten von -3 ist gut (r = 0,89) und deutet auf eine im wesentlichen kugelige Form des durch den Diffusor belichteten Raumes.

**Figuren 5 und 6** zeigen zwei weitere, beispielhafte Diffusorrohlinge 1, aus denen nach dem beschriebenen Verfahren Diffusoren für verschiedene Anwendungen herstellbar sind. Der Diffusorrohling 1 gemäss Figur 5 weist ein spitzes, distales Ende 1.1 auf und der distale Bereich seiner Mantelfläche ist halb um den Umfang mit Energierichtungsgebem 21 (z.B. axial verlaufende Rippen) versehen, derart, dass nur in diesem Oberflächenbereich eine lichtstreuende Struktur erstellt werden kann. Ein solcher Diffusorrohling ergibt einen Diffusor mit einem Wirkungsbereich, der etwa die Form einer Halbkugel aufeist. Der Diffusorrohling 1 gemäss Figur 6 weist ein stumpfes distales Ende 1.1 auf und ein mittlerer Bereich seiner Umfangsfläche ist halb um den Umfang mit Energierichtungsgebem 21 (z.B. Höcker) versehen. Ein solcher Diffusorrohling ergibt mit dem in Figur 1 illustrierten Verfahren einen Diffusor mit einem Wirkungsbereich, der etwa einem halben Kreiszylinder entspricht.

Aus Diffusorrohlingen, analog zu denen, die in den Figuren 5 und 6 dargestellt sind, lassen sich Diffusoren mit verschiedenst geformten Wirkungsbereichen erstellen. Dabei brauchen die Diffusorrohlinge nicht gezwungenermassen wie dargestellt stiftförmig zu sein und kreisrunde Querschnitte aufzuweisen. Sie können auch eine mehr gedrungene Form haben, konisch ausgestaltet sein und/oder polygone oder unregelmässige Querschnitte aufweisen.

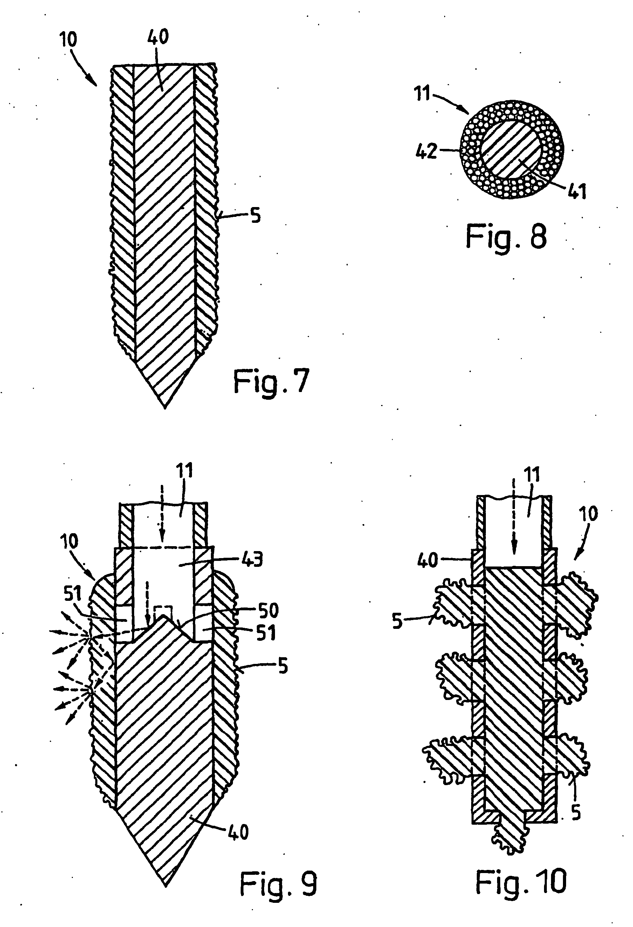

**Figur 7** zeigt in einem axialen Schnitt einen weiteren Diffusor 10 mit einem Diffusorkern 40, wobei der Diffusorkern 40 für weitere, beispielsweise nicht-optische Funktionen ausgerüstet ist. Das die lichtstreuende Oberflächenstruktur tragende Diffusormaterial (z.B. Polymerpin) ist am Diffusorkern 40 perifer angeordnet und deckt die Oberfläche des Diffusorkerns 40 ganz oder teilweise. Der Diffusorkem 40 besteht beispielsweise aus Titan und übernimmt in einem Diffusorimplantat beispielsweise eine tragende Funktion. Der Diffusor kann in situ oder ex situ aus einem entsprechenden Diffusorrohling hergestellt werden.

In den Diffusor 10 gemäss Figur 7 ist Licht nur über einen Teilbereich der proximalen Stirnfläche (äusserer Ring) einzukoppeln. Dafür kann beispielsweise ein Lichtleiter 11 verwendet werden, wie er im Querschnitt in der Figur 8 dargestellt ist. Dieser Lichtleiter 11 weist einen Leiterkem 41 auf und darum herum angeordnete, lichtleitende Fasern 42, wobei der Querschnitt des Leiterkerns 41 auf den proximalen Querschnitt des Diffusorkerns 40 abgestimmt ist.

Der Diffusorkem 40 kann anstelle der genannten tragenden Funktion oder zusätzlich dazu weitere Funktionen haben und dazu aus einem entsprechenden Material bestehen. Solche weitere Funktionen für einen ex situ erstellten Diffusor ist beispielsweise eine die Bewegung des Diffusors beim Einführen an eine zu belichtende Stelle kontrollierende Funktion, oder, wenn der Diffusor bzw. die Diffusorkappe als Instrument ausgebildet ist (siehe Figuren 11 und 12), eine Spülungs- oder Absaugfunktion, wofür der Diffusorkern dann als hohles Kondukt ausgebildet ist. In einem derartigen, hohlen Kondukt in einem Diffusorkem eines beispielsweise in situ erstellten Diffusors können auch weitere Lichtleiter verlaufen, die beispielsweise eine Aufnahmefunktion haben und an eine Mikrokamera angeschlossen sind, was beispielsweise zur gleichzeitigen Analyse eines Belichtungseffektes oder zur Detektion und Lokalisierung von fluoreszenzmarkierten Tumorzellen dienen kann.

Der Diffusorkem 40 eines in situ erstellten Diffusors (Diffusorimplantat) kann auch eine Freisetzungsfunktion haben, derart, dass er ein Medikament an das den Diffusor umgebende Gewebe abgeben kann. Bei der Verwendung von resorbierbaren Polymeren oder Gelen als Diffusormaterial kann diese Freisetzungsfunktion auch über das Diffusormaterial direkt erfolgen. Der Diffusorkern kann auch als vom Diffusor getrenntes, optisches Element ausgestaltet sein und zur Einkoppelung von Licht einer weiteren Wellenlänge (z.B. zur Aktivierung eines weiteren lichtempfindlichen Medikamentes) ausgerüstet sein oder zur Einkoppelung von infrarotem Licht zur Erwärmung des den Diffusor umgebenden Gewebes. Die Anordnung des Diffusormaterials auf dem Diffusorkern 40 ist an die Funktion des Diffusorkerns 40 anzupassen.

**Figur 9** zeigt in einem axialen Schnitt einen weiteren Diffusor, der in situ oder ex situ erstellbar ist und der wiederum einen Diffusorkem 40 aufweist, an dem das Diffusormaterial beispielsweise als Beschichtung angeordnet ist. Für die Ankoppelung des Lichtleiters 11 weist der Diffusorkern 40 einen proximalen Bereich mit einer zentralen Öffnung 43 auf, wobei auf dem Öffnungsgrund eine beispielsweise kegelförmige Spiegelfläche 50 angeordnet ist und über der Spiegelfläche Lichtaustrittsöffnungen 51. Ein distales Ende eines Lichtleiters 11 (ohne Umhüllung und mit vorteilhafterweise an die Spiegelfläche 50 angepasster Stirnfläche) wird zur Einkoppelung des Lichtes in die zentrale Öffnung 43 eingeführt. Das durch den Lichtleiter 11 zugeführte Licht wird von der Spiegelfläche 50 reflektiert und erreicht durch die Lichtaustrittsöffnungen 51 das Diffusormaterial, wie dies in der Figur 9 mit Pfeilen angedeutet ist.

**Figur 10** zeigt, wiederum in einem axialen Schnitt, einen weiteren Diffusor 10 mit einem Diffusorkem 40, wobei auch dieser Diffusor in situ oder ex situ erstellt werden kann. Der Diffusorkern 40 ist hülsenförmig und weist durchgehende Öffnungen auf. Das Diffusormaterial, das beispielsweise ein thermoplastisches Polymer, Gel oder aushärtbares Polymer ist, ist im entsprechenden Diffusorrohling im Innern des hülsenförmigen Diffusorkerns 40 vorgelegt und wird bei der Erstellung des Diffusors 10, bei welcher das Diffusormaterial mit mechanischen Vibrationen tiefer in den Diffusorkern gepresst wird, aus den Öffnungen in das umliegende Knochengewebe oder künstliche, poröse Formmaterial gepresst und erhält damit eine lichtstreuende Oberflächenstruktur 5.

**Figuren 11 und 12** zeigen Diffusoren 10, die ex situ erstellt werden und eine Diffusorkappe 14 aufweisen, die die Form von Instrumenten, bzw. Instrumententeilen aufweisen. Das in Figur 11 dargestellte Instrument ist ein längs geschnitten dargestelltes Skalpell, dessen Klinge als Diffusorkappe 12 ausgebildet ist, das heisst einen Diffusor 10 enthält. Die Diffusorkappe besteht beispielsweise aus einem transparenten Keramikmaterial, das vorteilhafterweise nur in jenen Bereichen relevant porös ist, in denen es als poröses Formmaterial dienen soll, während es insbesondere im Bereich der Schneidekante möglichst kompakt ist. Eine Kopplungsstelle für einen Lichtleiter (nicht dargestellt) ist im Bereich des Griffstückes 60 angeordnet. Durch Einkoppelung von Licht in den Diffusor 10 wird die Skalpellklinge leuchtend und kann ihr eigenes Arbeitsfeld homogen beleuchten.

Die Skalpellklinge gemäss Figur 11 wird beispielsweise hergestellt, indem ein flüssiges Diffusormaterial in eine entsprechende Bohrung der vorbereiteten Klinge eingesaugt oder mit den vorgängig beschriebenen, anderen Methoden eingebracht wird. Die Klinge kann nach der Herstellung des Diffusors 10 auch noch nachbearbeitet werden. Damit das Diffusormaterial im Bereich des Griffstückes 60 keine lichtstreuende Oberfläche erhält, ist dort kein poröses Formmaterial sondern ein kompaktes Formmaterial vorzusehen.

Es ist aber auch möglich, im Bereich des Griffstückes 60 eine etwas grössere Bohrung vorzusehen als im Bereich der Klinge und das Diffusormaterial in Form eines Stiftes in das Griffstück einzuführen und mit Ultraschall weiter in die Klinge einzupressen und das Licht über den Griff als Lichtleiter in die Klinge weiterzuleiten.

**Figur 12** zeigt als weiteres Beispiel für einen Diffusor 10 mit Diffusorkappe 14 ein scheren- oder zangenartiges Instrument, dessen Klingen oder Zangenarme 70 und 71 in der für die Skalpellklinge von Figur 11 beschriebenen Weise mit je einem Diffusor (schematisch als gestrichelte Linie angedeutet) ausgerüstet sind. Bei der Verwendung des Instrumentes dienen die Klingen oder Zangenarme 70 und 71 gleichzeitig als Quelle diffusen Lichtes, mit dem das Arbeitsfeld des Instrumentes gleichmässig beleuchtet werden kann.

Die beschriebenen Diffusoren, die mit dem dargestellten Verfahren aus den beschriebenen Diffusorrohlingen hergestellt sein können, werden bspw. für photodynamische Therapieverfahren verwendet, insbesondere für die Behandlung von Tumor-Erkrankungen. Beim hier beschriebenen Verfahren zum Einringen von diffusem Licht in einen Gewebebereich, bei welchem einer der beschriebenen und beanspruchten, insbesondere stiftförmigen, Diffusorrohlinge ins Gewebe implantiert wird, ist dann das Gewebe bspw. ein Knochengewebe und der zu behandelnde Knochengewebebereich der Bereich eines Knochentumors oder einer Metastase.

Das photodynamische Therapieverfahren beinhaltet demnach die Verfahrensschritte Einbringen einer lichtempfindlichen Substanz ins Tumorgewebe oder die Metastase, Herstellen eines Diffusors gemäss einer Ausführungsform des hier beschriebenen und beanspruchten Verfahrens (in situ) oder Einbringen eines ex situ hergestellten Diffusors ins Tumorgewebe oder die Metastase, Belichten des Tumorgewebes/der Metastase durch den Diffusor, insbesondere mit einer spezifischen Wellenlänge, wodurch die lichtempfindliche Substanz aktiviert und eine chemische Reaktion augelöst wird, durch die die Tumorzellen bzw. die Metastase vernichtet werden. Die Verfahrensschritte "Einbringen der Substanz" und "Herstellen des Diffusors" können auch in umgekehrter Reihenfolge erfolgen. Das Belichten muss nicht mit Licht im sichtbaren Wellenlängenbereich erfolgen, der Begriff "belichten" erstreckt sich auch auf Bestrahlen mit elektromagnetischer Strahlung anderer Wellenlängen, insbesondere im Infrarot- oder Ultraviolettbereich.

Das Einbringen der lichtempfindlichen Substanz kann dadurch geschehen, dass dem Patienten eine Substanz verabreicht wird, die sich vornehmlich im Tumorgewebe/der Metastase ansammelt. Die Substanz kann aber auch lokal in das Tumorgewebe oder die Metastase appliziert werden. Eine Möglichkeit, dies zu tun, ist wie erwähnt das Freisetzen mittels des Diffusors oder des Diffusorrohlings.

## Patentansprüche

1. Anordnung
- mit einem Lichtdiffusor (10), der aus einem mindestens teilweise transparenten Diffusormaterial besteht, der ein proximales Ende aufweist, das für die Einkoppelung von durch einen Lichtleiter (11) zugeführtem Licht in den Lichtdiffusor (10) ausgerüstet ist, und der lichtstreuende Oberflächenbereiche aufweist, durch die das eingekoppelte Licht diffus aus dem Lichtdiffusor (10) ausgelenkt wird, wobei die lichtstreuenden Oberflächenbereiche Strukturen (5) aufweisen, die durch Interpenetration des flüssigen Diffusormaterials in eine Grenzschicht (4) eines porösen Formmaterials (2) und anschliessendes Bringen des Diffusormaterials in einen festen oder gelförmigen Zustand entstanden und dadurch oberflächenspannungsbedingt und hinterschnitten sind,
- und mit dem Lichtleiter (11) oder einer Lichtquelle, der bzw. die an den Lichtdiffusor angekoppelt ist

2. Anordnung nach Anspruch 1, **dadurch gekennzeichnet, dass** der Lichtdiffusor stiftförtnig ist und die lichtstreuenden Oberflächenbereiche auf seiner Mantelfläche und/oder an seinem distalen Ende (1.1) angeordnet sind.

3. Anordnung nach einem der Ansprüche 1 oder 2, **dadurch gekennzeichnet, dass** das Diffusormaterial ein thermoplastisches Material, ein durch chemische Reaktion aushärtbares Material, oder ein Gel ist.

4. Anordnung nach einem der Ansprüche 1 bis 3, **dadurch gekennzeichnet, dass** der Lichtdiffusor einen Diffusorkern (40) aufweist, der für eine weitere Funktion ausgerüstet ist.

5. Anordnung nach Anspruch 4, **dadurch gekennzeichnet, dass** der Diffusorkern (40) aus einem nicht transparenten Material besteht und für eine nicht-optische Funktion ausgerüstet ist.

6. Anordnung nach Anspruch 4, **dadurch gekennzeichnet, dass** der Diffusorkern (40) mindestens teilweise aus einem transparenten Material besteht und für eine weitere, optische Funktion ausgerüstet ist.

7. Anordnung nach einem der Ansprüche 1 bis 6, **dadurch gekennzeichnet, dass** der Lichtdiffusor in Knochengewebe implantiert ist und das Knochengewebe das Formmaterial darstellt.

8. Anordnung nach einem der Ansprüche 1 bis 6, **dadurch gekennzeichnet, dass** der Lichtdiffusor eine Diffusorkappe (14) aus dem porösen Formmaterial (2) aufweist, wobei das poröse Formmaterial (2) der Diffusorkappe (14) in einer Grenzschicht (4) durch das Diffusormaterial interpenetriert ist

9. Anordnung nach Anspruch 8, **dadurch gekennzeichnet, dass** die Diffusorkappe (14) als Instrument oder Instrumententeil ausgebildet ist.

10. Anordnung nach einem der Ansprüche 8 oder 9, **dadurch gekennzeichnet, dass** die Diffusorkappe eine nicht homogene Porosität aufweist.

11. Verfahren zur Herstellung einer Anordnung nach einem der Ansprüche 1 bis 6 oder 8 bis 10, **dadurch gekennzeichnet, dass** das Diffusormaterial in flüssigem Zustand in einem Grenzbereich in Poren des porösen Formmaterials (2) gepresst und dann in einen festen oder gelförmigen Zustand gebracht wird und an einen Lichtleiter (11) oder eine Lichtquelle angekoppelt wird wobei das poröse Formmaterial ein Künstliches ist.

12. Verfahren nach Anspruch 11, **dadurch gekennzeichnet, dass** das poröse Formmaterial (2) als Diffusorkappe (14) am Diffusor (10) belassen wird.

13. Verfahren nach Anspruch 11, **dadurch gekennzeichnet, dass** das poröse Formmaterial (2) vom Diffusor (10) entfernt wird.

14. Verfahren nach einem der Ansprüche 11 bis 13, **dadurch gekennzeichnet, dass** ein Diffusorrohling (1), der aus einem thermoplastischen Diffusormaterial besteht, ein distales Ende (1.1) und ein für die Einkoppelung des zugeführten Lichts ausgerüstetes, proximales Ende (1.2) aufweist, in oder auf dem porösen Formmaterial (2) positioniert wird und dass das proximale Ende (1.2) mit mechanischen Vibrationen beaufschlagt und gleichzeitig der Diffusorrohling (1) gegen das poröse Formmaterial (2) gepresst wird, derart, dass sich das thermoplastische Diffusormaterial in Oberflächenbereichen, die mit dem porösen Formmaterial (2) in Kontakt stehen, verflüssigt und in das poröse Formmaterial (2) eingepresst werden.

15. Verfahren nach einem der Ansprüche 11 bis 14, **dadurch gekennzeichnet, dass** das poröse Formmaterial (2) eine Porosität mit einer Porengrösse im Bereiche von 0,005 bis 1,0 mm aufweist.

16. Verfahren nach einem der Ansprüche 11 bis 15, **dadurch gekennzeichnet, dass** in dem porösen Formmaterial (2) eine Öffnung (3) erstellt wird und dass der Diffusorrohling (1) in der Öffnung (3) derart positioniert wird, dass sein distales Ende (1.1) weder bei der Positionierung noch bei der Beaufschlagung und Pressung mit einem Öffnungsgrund in Kontakt kommt.

17. Verfahren nach einem der Ansprüche 11 bis 15, **dadurch gekennzeichnet, dass** in dem porösen Formmaterial (2) eine Öffnung oder keine Öffnung erstellt wird und dass der Diffusorrohling (1) mit dem distalen Ende (1.1) voran in das poröse Formmaterial (2) oder einen Öffnungsgrund getrieben wird.

18. Diffusorrohling (1) zur Anwendung in dem Verfahren nach einem der Ansprüche 16 oder 17 welcher stiftförmig ist und mindestens teilweise aus einem transparenten, thermoplastischen Diffusormaterial besteht und ein distales und ein proximales Ende (1.1, 1.2) aufweist, wobei das proximale Ende (1.2) für die Einkoppelung zugeführten Lichts und für die Beaufschlagung mit mechanischen Vibrationen ausgerüstet ist, **dadurch gekennzeichnet, dass** er ferner auf seiner Mantelfläche angeordnete Oberflächenbereiche aufweist, an denen das Diffusormaterial für die Erzeugung einer lichtstreuenden Oberflächenstruktur (5) verflüssigbar und in das poröse Formmaterial (2) pressbar ist und die zu diesem Zwecke über weitere Oberflächenbereiche vorstehen oder mit vorstehenden Energierichtungsgebem (21) ausgerüstet sind und die am Diffusorrohling (1) derart angeordnet sind, dass sie mit dem porösen Formmaterial (2) in Kontakt bringbar sind.

19. Diffusorrohling nach Anspruch 18, **dadurch gekennzeichnet, dass** die weiteren Oberflächenbereiche poliert oder beschichtet sind.

20. Diffusorrohling nach einem der Ansprüche 18 bis 19, **dadurch gekennzeichnet, dass** er für eine Verwendung als Belichtungsimplantat geeignet ist und aus einem klinisch anwendbaren, thermoplastischen Polymer besteht.

21. Diffusorrohling nach Anspruch 20, **dadurch gekennzeichnet, dass** das Polymer biologisch resorbierbar ist.

22. Diffusorrohling nach einem der Ansprüche 18 bis 21, **dadurch gekennzeichnet, dass** er einen Diffusorkern (40) aufweist der für weitere Funktionen ausgerüstet ist, wobei das Diffusormaterial peripher am Diffusorkern (40) analeordnet ist oder wobei das Diffüsonnaterial im Innern des Diffusorkerns (40) vorgelegt ist und der Diffusorkern Öffnungen aufweist, durch die das Diffusormaterial an die Oberfläche des Diffusorkerns (40) pressbar ist.

## Claims

1. Device
- with a light diffuser (10) consisting of an at least partly transparent diffuser material, the light diffuser comprising a proximal end suitable for coupling light supplied by a light conductor (11) into the light diffuser (10), and the light diffuser further comprising light scattering surface areas through which the light coupled into the light diffuser is deflected diffusely out of the light diffuser (10), **characterized in that** the light scattering surface areas comprise structures (5) which are produced by interpenetration of the diffuser material in a liquid state in a boundary layer (4) of a porous shaping material (2) and which therefore comprise undercut forms induced by a surface tension,
- and with the light conductor (11) or a light source being coupled to the light diffuser.

2. Device according to claim 1, **characterized in that** it is pin-shaped and the light scattering surface areas are situated upon its circumferential surface and/or at its distal end (1.1).

3. Device according to any one of claims 1 or 2, **characterized in that** the diffuser material is a thermoplastic material, a material which is thermosetting through a chemical reaction, or a gel.

4. Device according to one of the claims 1 to 3, **characterized in that** it comprises a diffuser core (40) equipped for a further function.

5. Device according to claim 4, **characterized in that** the diffuser core (40) consists of a non-transparent material and is equipped for a non-optical function.

6. Device according to claim 4, **characterized in that** the diffuser core (40) consists at least partly of a transparent material and is equipped for a further optical function.

7. Device according to any one of claims 1 to 6, **characterized in that** it is implanted in bone tissue and the bone tissue represents the shaping material.

8. Device according to any one of claims 1 to 6, **characterized in that** it comprises a diffuser cap (14) of the porous shaping material (2), wherein the porous shaping material (2) of the diffuser cap (14) is interpenetrated in a boundary layer (4) by the diffuser material.

9. Device according to claim 8, **characterized in that** the diffuser cap (14) is designed as an instrument or part of an instrument.

10. Device according to any one of claims 8 or 9, **characterized in that** the diffuser cap comprises a non-homogenous porosity.

11. Method for producing a deviceaccording to any one of claims 1 to 10, **characterized in that** the diffuser material in a liquid state is pressed in a boundary region into pores of the porous shaping material (2) and is then brought into a solid or gellike state and is coupled to a light conductor (11) or a light source.

12. Method according to claim 11, **characterized in that** the porous shaping material (2) is left on the diffuser (10) as a diffuser cap (14).

13. Method according to claim 11, **characterized in that** the porous shaping material (2) is removed from the diffuser (10).

14. Method according to any one of claims 11 to 13, **characterized in that** a diffuser blank (1) consisting of a thermoplastic diffuser material comprises a distal end (1.1) and a proximal end (1.2), which is equipped for coupling the supplied light into the light diffuser, is positioned in or on the porous shaping material (2) and that mechanical vibration is applied to the proximal end (1.2), and the diffuser blank (1) is simultaneously pressed against the porous shaping material (2), so that the thermoplastic diffuser material liquefies in those regions of the surface area in contact with the porous shaping material (2) and is pressed into the porous shaping material (2).

15. Method according to any one of claims 11 to 14, **characterized in that** the porous shaping material (2) comprises a porosity with a pore size ranging between 0,005 and 1.0 mm.

16. Method according to any one of claims 11 to 15, **characterized in that** an opening (3) is provided in the porous shaping material (2) and that the diffuser blank (1) is positioned in the opening (3) such that its distal end (1.1) is not brought in contact with the bottom of the opening, neither when it is positioned nor when it is pressed and the mechanical vibration is applied.

17. Method according to any one of claims 11 to 15, **characterized in that** an opening is provided in the porous shaping material (2) and the diffuser blank (1) is driven into a bottom of the opening, the distal end (1.1) facing forward or that no opening is provided in the porous shaping material and the diffuser blank is driven into the porous shaping material (2) with the distal end facing forward.

18. Diffuser (1) blank suitable for being used in the method according to any one of claims 16 to 17, which is pin-shaped and it consists at least partly of a transparent, thermoplastic diffuser material and comprises a distal end and a proximal end (1.1, 1.2), wherein the proximal end (1.2) is suitable for coupling light into the diffuser blank and for mechanical vibrations to be applied to the diffuser blank, **characterized in that** it further comprises surface areas arranged on its surface, where the diffuser material is to be liquefiable for the generation of a light scattering surface structure (5) and to be pressable into the porous shaping material (2), and which to this purpose protrude beyond further surface areas or are furnished with protruding energy directors (21) and are situated on the diffuser blank (1) in such a manner that they are able to be brought into contact with the porous shaping material (2).

19. Diffuser blank according to claim 18, **characterized in that** the further surface areas are polished or coated.

20. Diffuser blank according to any one of claims 18 to 19, **characterized in that** it is suitable as an illuminative implant and consists of a clinically applicable, thermoplastic polymer.

21. Diffuser blank according to claim 20, **characterized in that** the polymer is biologically resorbable.

22. Diffuser blank according to any one of claims 18 to 21, **characterized in that** it comprises a diffuser core (40) equipped for further functions, wherein the diffuser material is situated at the periphery of the diffuser core (40) or wherein the diffuser material is provided inside the diffuser core (40) and the diffuser core comprises openings, through which the diffuser material can be pressed to the surface of the diffuser core (40).

## Revendications

1. Arrangement
avec un diffuseur de lumière (10) constitué d'un matériau diffuseur au moins partiellement transparent et présentant une extrémité proximale adaptée pour injecter dans le diffuseur de lumière (10) de la lumière apportée par un conducteur de lumière (11) ainsi que des parties de surface qui diffusent la lumière et par lesquelles la lumière injectée sort du diffuseur de lumière (10) de manière diffusée,
**caractérisé en ce que**
les parties de surface qui diffusent de la lumière présentent des structures (5) formées par pénétration du matériau liquide du diffuseur dans une couche frontière (4) d'un matériau poreux de moulage (2) et amenées en contre-dépouille sous l'action de la tension superficielle,
- et avec le conducteur de lumière (11) ou une source de lumière qui est accroché à le diffuseur de lumière.

2. Arrangementselon la revendication 1, **caractérisé en ce qu'**il a la forme d'une tige et **en ce que** les parties de surface qui diffusent la lumière sont disposées sur sa surface d'enveloppe et/ou à son extrémité distale (1.1).

3. Arrangementselon l'une des revendications 1 ou 2, **caractérisé en ce que** le matériau diffuseur est un matériau thermoplastique, un matériau durcissable par réaction chimique ou un gel.

4. Arrangementselon l'une des revendications 1 à 3, **caractérisé en ce qu'**il présente une âme de diffuseur (40) adaptée pour assurer une autre fonction.

5. Arrangementselon la revendication 4, **caractérisé en ce que** l'âme (40) du diffuseur est constituée d'un matériau non transparent et est adaptée pour assurer une fonction autre qu'optique.

6. Arrangement la revendication 4, **caractérisé en ce que** l'âme (40) du diffuseur est constituée au moins en partie d'un matériau transparent et est adaptée pour assurer une autre fonction optique.

7. Arrangementselon l'une des revendications 1 à 6, **caractérisé en ce qu'**il est implanté dans le tissu osseux et **en ce que** le tissu osseux forme le matériau de moulage.

8. Arrangementselon l'une des revendications 1 à 6, **caractérisé en ce qu'**il présente un capuchon de diffuseur (14) constitué du matériau poreux de moulage (2) et **en ce qu'**une couche frontière (4) du matériau poreux de moulage (2) du capuchon (14) du diffuseur est imprégnée par le matériau diffuseur.

9. Arrangementselon la revendication 8, **caractérisé en ce que** le capuchon (14) du diffuseur est configuré comme instrument ou partie d'instrument.

10. Arrangementselon l'une des revendications 8 ou 9, **caractérisé en ce que** la porosité du capuchon du diffuseur n'est pas homogène.

11. Procédé de fabrication d'un arrangement selon l'une des revendications 1 à 10, **caractérisé en ce que** le matériau diffuseur est refoulé à l'état liquide dans les pores d'une zone frontière du matériau poreux de moulage (2) et est ensuite amené à l'état solide ou de gel et est accroché a un conducteur de lumière (11) ou une source de lumière.

12. Procédé selon la revendication 11, **caractérisé en ce que** le matériau poreux de moulage (2) est conservé comme capuchon (14) sur le diffuseur (10).

13. Procédé selon la revendication 11, **caractérisé en ce que** le matériau poreux de moulage (2) est enlevé du diffuseur (10).

14. Procédé selon l'une des revendications 11 à 13, **caractérisé en ce qu'**une ébauche de diffuseur (1) constituée d'un matériau diffuseur thermoplastique et présentant une extrémité distale (1.1) et une extrémité proximale (1.2) adaptée pour permettre l'injection de la lumière apportée est placée dans ou sur le matériau poreux de moulage (2), **en ce que** des vibrations mécaniques sont appliquées sur l'extrémité proximale (1.2), **en ce que** l'ébauche de diffuseur (1) est en même temps refoulée contre le matériau de moulage (2) de telle sorte que le matériau diffuseur thermoplastique se liquéfie dans les parties de la surface en contact avec le matériau poreux de moulage (2) et soit refoulé dans le matériau poreux de moulage (2).

15. Procédé selon l'une des revendications 11 à 14, **caractérisé en ce que** la porosité du matériau poreux de moulage (2) est constituée de pores dont la taille est comprise dans la plage de 0,005 à 1,0 mm.

16. Procédé selon l'une des revendications 11 à 15, **caractérisé en ce qu'**une ouverture (3) est formée dans le matériau poreux de moulage (2) et **en ce que** l'ébauche de diffuseur (1) est placée dans l'ouverture (3) de telle sorte que son extrémité distale (1.1) n'entre en contact avec le fond de l'ouverture ni pendant le positionnement, ni pendant l'application de vibrations ni pendant le refoulement.

17. Procédé selon l'une des revendications 11 à 15, **caractérisé en ce qu'**une ouverture est formée ou non dans le matériau poreux de moulage (2) et **en ce que** l'ébauche de diffuseur (1) est avancée par son extrémité distale (1.1) dans le matériau poreux de moulage (2) ou dans le fond de l'ouverture.

18. Ebauche de diffuseur (1) destinée à être utilisée dans le procédé selon l'une des revendications 16 ou 17, en forme de tige, constituée au moins en partie d'un matériau diffuseur thermoplastique transparent et présentant une extrémité distale et une extrémité proximale (1.1, 1.2), l'extrémité proximale (1.2) étant adaptée pour permettre l'injection de la lumière apportée et pour appliquer des vibrations mécaniques, **caractérisée en ce que**
à l'extrémité distale de sa surface d'enveloppe, elle présente en outre des parties de surface dans lesquelles le matériau diffuseur peut être liquéfié pour former une structure de surface (5) diffusant la lumière et peut être refoulé dans le matériau poreux de moulage (2), lesquelles parties de surface débordent dans ce but au-delà d'autres parties de surface ou sont dotées de dispositifs (21) en saillie d'orientation de l'énergie et sont disposées sur l'ébauche de diffuseur (1) de manière à pouvoir être amenées en contact avec le matériau poreux de moulage (2).

19. Ebauche de diffuseur selon la revendication 18, **caractérisée en ce que** les autres parties de surface sont polies ou revêtues.

20. Ebauche de diffuseur selon l'une des revendications 18 et 19, **caractérisée en ce qu'**elle convient pour être utilisée comme implant d'éclairage et **en ce qu'**elle est constituée d'un polymère thermoplastique utilisable cliniquement.

21. Ebauche de diffuseur selon la revendication 20, **caractérisée en ce que** le polymère est biologiquement résorbable.

22. Ebauche de diffuseur selon l'une des revendications 18 à 21, **caractérisée en ce qu'**elle présente une âme de diffuseur (40) adaptée pour assurer d'autres fonctions, le matériau diffuseur étant disposé à la périphérie de l'âme (40) du diffuseur ou le matériau diffuseur étant placé préalablement à l'intérieur de l'âme (40) du diffuseur, l'âme du diffuseur présentant des ouvertures par lesquelles le matériau diffuseur peut être refoulé contre la surface de l'âme (40) du diffuseur.
